# EUROPÄISCHE PATENTANMELDUNG

(11) **EP 3 756 725 A1**
(43) Veröffentlichungstag der Anmeldung: **30.12.2020**
(21) Anmeldenummer: 19183189.0
(22) Anmeldetag: 28.06.2019
(51) Int. Cl.: A61N 1/08, A61N 1/05

(54) **IMPLANTIERBARE ELKTRODENLEITUNG MIT ZU EINEM GEFLECHTVERBUNDENEN LEITERN**

(71) Anmelder: BIOTRONIK SE & Co. KG, 12359 Berlin (DE)
(72) Erfinder: Richter, Jan Helge, 12355 Berlin (DE)
(74) Vertreter: Krämer, Thomas

(57) **Zusammenfassung**

Eine implantierbare Elektrodenleitung (1) umfasst zumindest einen Elektrodenpol (130) und eine Mehrzahl von elektrischen Leitern (121-124), von denen zumindest einer mit dem zumindest einen Elektrodenpol (130) elektrisch verbunden ist. Die Mehrzahl von Leitern (121-124) ist zu einem entlang einer Längsachse (A) erstreckten Geflecht (12) miteinander verbunden, wobei zumindest ein erster Leiter (121, 122) der Mehrzahl von Leitern (121-124) wendelförmig in eine erste Drehrichtung (D1) um die Längsachse (A) und zumindest ein zweiter Leiter (123, 124) der Mehrzahl von Leitern (121-124) wendelförmig in eine der ersten Drehrichtung (D1) entgegen gesetzte, zweite Drehrichtung (D2) um die Längsachse (A) gewunden ist.

## Beschreibung

Die Erfindung betrifft eine implantierbare Elektrodenleitung nach dem Oberbegriff des Anspruchs 1 sowie ein Verfahren zum Herstellen einer implantierbaren Elektrodenleitung.

Eine derartige implantierbare Elektrodenleitung kann an ein aktives elektrisches Gerät, zum Beispiel einen Herzschrittmacher oder einen Neurostimulator, angeschlossen werden und beispielsweise als Herzelektrodenleitung im Herzen oder als Neuroelektrodenleitung im Rückenmark oder auch im Gehirn eines Patienten implantiert werden. Über eine solche Elektrodenleitung und ein daran angeschlossenes aktives Gerät können elektrische Signale zur Stimulation an den Patienten abgegeben werden.

Eine derartige implantierbare Elektrodenleitung umfasst zumindest einen Elektrodenpol und eine Mehrzahl von elektrischen Leitern, von denen zumindest einer mit dem zumindest einen Elektrodenpol elektrisch verbunden ist.

Eine solche Elektrodenleitung ist dafür ausgelegt, nach ihrer Implantation üblicherweise über einen längeren Zeitraum im Körper des Patienten zu verbleiben. Eine solche Elektrodenleitung soll hierbei Untersuchungen am Patienten, insbesondere MRT (Magnetresonanztomographie) -Untersuchungen, zulassen, was heißt, dass ein bei einer MRT-Untersuchung erzeugtes elektromagnetisches Feld nicht zu einer Erwärmung an einem Leiter oder einem Elektrodenpol der Elektrodenleitung führen darf, die gegebenenfalls schädlich für den Patienten ist.

Eine Erwärmung an einer in einem Patienten implantierten Elektrodenleitung kann unter Umständen durch Einkoppeln elektromagnetischer Felder bewirkt werden. Die Kopplung der Elektrodenleitung mit dem elektromagnetischen Feld eines MR-Tomographen (der ein Anregungsfeld mit einer von der Magnetfeldstärke abhängigen Anregungsfrequenz, bei 1,5 Tesla zum Beispiel bei ca. 64 MHz, erzeugt) hängt von der effektiven Leitungslänge von zum Beispiel als Zuleitungen für Elektrodenpole dienenden Leitern der Elektrodenleitung ab. Befindet sich die effektive Leitungslänge der Elektrodenleitung im Bereich einer (Serien-)Resonanzfrequenz des elektromagnetischen Feldes, so können elektromagnetische Felder in die Elektrodenleitung einkoppeln und zu einer Erwärmung an der Elektrodenleitung führen, was es nach Möglichkeit zu vermeiden gilt.

Eine Elektrodenleitung soll generell insbesondere für die Neurostimulation, dünn ausgebildet sein. Zudem bestehen Vorgaben hinsichtlich der Länge der Zuleitungen und des maximalen ohmschen Widerstands.

Aus der US 2009/0259281 A1 ist eine implantierbare Elektrodenleitung bekannt, die einen ersten, inneren Leiter sowie einen außerhalb des inneren Leiters erstreckten, äußeren Leiter aufweist.

Bei einer aus der US 2015/0170792 A1 bekannten Elektrodeneinrichtung sind Leiter wendelförmig angeordnet. Ein innerer Leiter ist hierbei mit einem Elektrodenpol elektrisch kontaktiert.

Bei einer aus der US 2008/0147155 A1 bekannten Elektrodenleitung sind elektrische Leiter mit Polymerfäden zu einem Geflecht miteinander verbunden. Die elektrischen Leiter sind hierbei in eine gemeinsame Drehrichtung wendelförmig gewunden.

Bei einer aus der US 2009/0099441 A1 bekannten Elektrodenleitung sind Leiter in einem Ausgangszustand mit biologisch abbaubaren Fasern verflochten, die sich bei Implantierung der Elektrodenleitung auflösen.

Generell kann ein elektrischer Leiter einer Elektrodenleitung durch sogenannte Stichleitungen derart in seiner effektiven elektrischen Länge verändert werden, dass elektromagnetische Energie bei einer bestimmten MR-Anregungsfrequenz nicht mehr effektiv in die Elektrodenleitung einkoppeln kann und somit es an einem elektrischen Leiter der Elektrodenleitung nicht zu einer übermäßigen Erwärmung bei einer MR-Untersuchung kommt. Hierbei ist jedoch zu beachten, dass solche Stichleitungen zur Veränderung der effektiven elektrischen Länge eines oder mehrerer elektrischer Leiter Platz erfordern, der innerhalb einer Elektrodenleitung unter Umständen nicht ohne weiteres zur Verfügung steht.

Es besteht ganz allgemein ein Bedürfnis nach einer Elektrodenleitung, die konstruktiv einfach zu fertigen ist und dabei in flexibler Weise mit Blick auf den Verlauf von elektrischen Leitern in oder an der Elektrodenleitung und zudem mit Blick auf eine MRT-Kompatibilität einsetzbar ist.

Aufgabe der vorliegenden Erfindung ist es, eine implantierbare Elektrodenleitung sowie ein Verfahren zur Herstellung einer implantierbaren Elektrodenleitung zur Verfügung zu stellen, die auf konstruktiv einfache Weise eine bauraumgünstige Verlegung von elektrischen Leitern sowie eine flexible Anpassbarkeit mit Blick auf eine MRT-Kompatibilität ermöglichen.

Diese Aufgabe wird durch einen Gegenstand mit den Merkmalen des Anspruchs 1 gelöst.

Demnach ist bei der implantierbaren Elektrodenleitung die Mehrzahl von Leitern zu einem entlang einer Längsachse erstreckten Geflecht miteinander verbunden, wobei zumindest ein erster Leiter der Mehrzahl von Leitern wendelförmig in eine erste Drehrichtung um die Längsachse und zumindest ein zweiter Leiter der Mehrzahl von Leitern wendelförmig in eine der ersten Drehrichtung entgegengesetzte, zweite Drehrichtung um die Längsachse gewunden ist.

Die Elektrodenleitung weist mehrere elektrische Leiter auf, die gemeinsam ein Geflecht ausbilden, das sich entlang der Elektrodenleitung, zum Beispiel entlang eines Innenschlauchs der Elektrodenleitung, erstreckt. Ein jeder elektrischer Leiter weist beispielsweise einen Kern und eine umgebende elektrische Isolierung auf, sodass der elektrische Leiter einen Strom führen kann, benachbarte Leiter aber gegeneinander elektrisch isoliert sind.

Das Geflecht kann sich beispielsweise mit einer schlauchförmigen Grundform entlang der Längsachse erstrecken, wobei die Längsachse einer zentralen Längsachse der Elektrodenleitung entsprechen und das Geflecht somit um ein zentrales Lumen der Elektrodenleitung herumerstreckt ist. Alternativ kann das Geflecht aber auch entlang eines exzentrischen Nebenlumens der Elektrodenleitung und umfänglich um eine diesem Nebenlumen zugeordnete Längsachse herum erstreckt sein. Das Geflecht stellt einen umfänglich geschlossenen Hohlkörper dar, der sich entlang der Länge der Elektrodenleitung erstreckt und durch die miteinander verflochtenen Leiter der Elektrodenleitung gebildet ist. Alternativ kann das Geflecht auch als Zopf ohne Lumen ausgebildet sein.

Durch die Verbindung der Leiter zu einem Geflechtkörper wird ein flexibler, biegbarer Leiterstrang geschaffen, der flexibel zur Verwendung an einer bestimmten Elektrodenleitung angepasst werden kann. Im Rahmen des Geflechts können grundsätzlich beliebig viele Leiter miteinander verflochten sein. Erhältliche Flechtmaschinen ermöglichen beispielsweise das gleichzeitige Flechten von mehreren Hundert (elektrisch leitenden) Drähten oder (nichtleitenden) Fasern, wobei Leiter zweilagig in einer Flechtebene oder auch in mehreren Ebenen übereinander geflochten werden können.

Die Verwendung eines solchen Geflechts ermöglicht eine flexible Anpassbarkeit der Elektrodenleitung zum Beispiel mit Blick auf eine MRT-Kompatibilität. So können einzelne Leiter zur Verbindung eines an einem distalen Ende der Elektrodenleitung angeordneten Elektrodenpols mit einem an einem proximalen Ende der Elektrodenleitung angeordneten elektrischen Kontaktelement zum Anschließen der Elektrodenleitung an ein aktives Gerät, zum Beispiel einen Herzschrittmacher oder ein Neurostimulationsgerät, verwendet werden. Andere Leiter können demgegenüber als sogenannte Stichleitungen zur Verlängerung der effektiven elektrischen Länge von Leitern verwendet werden, die zur Verbindung eines Kontaktelements mit einem zugeordneten Elektrodenpol dienen. Die Leiter können hierbei in gewünschter Weise miteinander kontaktiert oder auch elektrisch aufgetrennt werden, sodass durch Anpassung des Geflechts eine flexibel konfigurierbare Leiteranordnung an der Elektrodenleitung geschaffen werden kann.

In einer Ausgestaltung weist das Geflecht eine definierte Länge auf, wobei die Mehrzahl von Leitern sich entlang der Länge des Geflechts erstrecken. Die das Geflecht ausbildenden Leiter weisen somit eine gemeinsame, einheitliche Länge auf, entsprechend der gesamten Länge des an der Elektrodenleitung verwendeten Geflechts. Leiter, die zur Verbindung eines Elektrodenpols mit einem zugeordneten Kontaktelement dienen, weisen somit grundsätzlich die gleiche Länge auf, was mit Blick auf eine MRT-Kompatibilität vorteilhaft sein kann.

Zur Ausbildung des Geflechts sind die Leiter miteinander verflochten, wobei das Geflecht über die Länge der Elektrodenleitung eine konstante Maschenweite oder auch eine variable Maschenweite aufweisen kann. Über die Wahl der Maschenweite kann die Länge der Leiter der Elektrodenleitung so vorgegeben werden, dass eine Einkopplung elektromagnetischer Energie bei einer vorbestimmten MR-Anregungsfrequenz nach Möglichkeit reduziert ist und die Elektrodenleitung somit eine vorteilhafte MRT-Kompatibilität aufweist.

In einer Ausgestaltung ist die Mehrzahl von Leitern an einem Innenschlauch angeordnet und um den Innenschlauch herum gewunden. Zur Herstellung der Elektrodenleitung können die Leiter beispielsweise um einen Kern, an dem der Innenschlauch angeordnet ist, herum geflochten werden, sodass sich ein schlauchförmiges Geflecht an dem Innenschlauch ergibt.

Der Innenschlauch definiert ein inneres Lumen der Elektrodenleitung. Der Innenschlauch kann hierbei beliebig gestaltet sein. Beispielsweise kann der Innenschlauch eine hydrophile Beschichtung aufweisen. In einer Ausgestaltung weist der Innenschlauch beispielsweise einen mehrschichtigen Aufbau aus Lagen unterschiedlicher Materialien auf.

Nach außen kann das Geflecht von Leitern durch einen Außenschlauch umgeben sein. Beispielsweise kann das Geflecht von Leitern mit einem Kunststoffmaterial umspritzt sein. Alternativ kann eine äußere Ummantelung durch einen sogenannten Reflowprozess hergestellt sein, im Rahmen dessen Schlauchabschnitte über das an dem Innenschlauch angeordnete Geflecht geschoben und durch Aufschmelzen miteinander verbunden werden.

Das Geflecht ist aus den Leitern der Elektrodenleitung gebildet, sodass die Leiter einen in seiner Grundform schlauchförmig erstreckten Geflechtkörper ausbilden, der sich entlang der Länge der Elektrodenleitung erstreckt. Die elektrischen Leiter sind hierbei wendeiförmig in unterschiedliche Drehrichtungen um die Längsachse, entlang derer sich das Geflecht erstreckt, gewunden und miteinander verflochten, sodass sich ein zusammenhängender Geflechtkörper ergibt. Erste Leiter erstrecken sich wendelförmig in eine erste Drehrichtung. Zweite Leiter erstrecken sich demgegenüber wendelförmig in eine der ersten Drehrichtung entgegengesetzte, zweite Drehrichtung, wobei die Leiter abwechselnd übereinander und untereinander zu liegen kommen und somit das zusammenhängende Geflecht ausbilden.

In einer Ausgestaltung ist der zumindest eine erste Leiter, der sich in die erste Drehrichtung um die Längsachse erstreckt, an einer ersten Verbindungstelle elektrisch mit dem zumindest einen Elektrodenpol verbunden. Ein solcher Leiter stellt somit eine elektrische Zuleitung für den Elektrodenpol dar.

An einer zweiten Verbindungstelle ist der zumindest eine erste Leiter, in einer Ausgestaltung, demgegenüber mit zumindest einem zweiten Leiter verbunden. Der zweite Leiter, der entgegengesetzt zum ersten Leiter in die zweite Drehrichtung um die Längsachse gewunden ist, kann auf diese Weise eine Stichleitung zur Verlängerung der effektiven elektrischen Länge des zugeordneten ersten Leiters verwirklichen, sodass die effektive elektrische Länge des als Zuleitung dienenden ersten Leiters so angepasst sein kann, dass eine Einkopplung elektromagnetischer Energie bei einer vorbestimmten MR-Anregungsfrequenz reduziert und eine übermäßige Erwärmung an der Elektrodenleitung bei einer MRT-Untersuchung damit verhindert ist.

Durch die elektrische Verbindung eines als Zuleitung dienenden, ersten Leiters mit einem als Stichleitung dienenden zweiten Leiter kann die elektrische Länge der Zuleitung verdoppelt werden, wobei auch möglich ist, den zweiten Leiter wiederum mit einem ersten Leiter zu verbinden, der ebenfalls als Stichleitung dient und somit eine zusätzliche Verlängerung der elektrischen Länge der Zuleitung ermöglicht.

Grundsätzlich ist die Einkopplung eines elektromagnetischen Felds bei einer vorbestimmten MR-Anregungsfrequenz - zum Beispiel ca. 64 MHz bei einer MR-Magnetfeldstärke von 1,5 Tesla und ca. 128 MHz bei einer MR-Magnetfeldstärke von 3 Tesla - in einen Leiter bei einer effektiven Leitungslänge, die einer Serienresonanz entspricht, maximal. Bei einer solchen Serienresonanz ist der Betrag der Impedanz minimal, und aufgrund maximaler Einkopplung des elektromagnetischen Feldes kann es zu einer Feldüberhöhung und damit einer vergleichsweise großen Erwärmung an der Elektrodenleitung kommen. Bei einer effektiven Leitungslänge eines Leiters, die einer Parallelresonanz entspricht, ist demgegenüber der Betrag der Impedanz an dem Leiter maximal, und entsprechend ist die Einkopplung des elektromagnetischen Felds unterdrückt. Wünschenswert ist somit, die effektive Leitungslänge der Leiter so einzustellen, dass diese einer Parallelresonanz entspricht.

Die Bestimmung, wann bei einer vorbestimmten MR-Anregungsfrequenz, zum Beispiel 64 MHz oder 128 MHz, eine Parallelresonanz auftritt, kann durch Computersimulationen oder auch messtechnisch anhand geeigneter Versuchsreihen erfolgen. Zum Beispiel kann messtechnisch das Impedanzspektrum für unterschiedliche Leitungslängen anhand des Reflexionskoeffizienten der Leiter bei Simulation von menschlichem Gewebe durch eine Kochsalzlösung bestimmt werden. Hieraus kann bei der vorbestimmten MR-Anregungsfrequenz eine vorteilhafte effektive Leitungslänge eines Leiters bestimmt werden, die einer Parallelresonanz entspricht. Anhand dieser so bestimmten effektiven Leitungslänge kann die Länge der Stichleitung für einen als Zuleitung dienenden Leiter dann so gewählt werden, dass die Summe aus der Stichleitungslänge und der Länge der Zuleitung der gewünschten effektiven Leitungslänge entspricht.

Die effektive Leitungslänge kann hierbei auf eine erste Parallelresonanz im Impedanzspektrum abgestimmt werden. Denkbar und möglich ist aber auch, die effektive Leitungslänge durch Verlängerung der Stichleitungslänge um eine halbe Wellenlänge (oder ein Vielfaches der halben Wellenlänge) auf eine Parallelresonanz höherer Ordnung abzustimmen.

Die Leiter des Geflechts können in beliebiger Weise miteinander verbunden und lokal aufgetrennt werden. Dass aus den Leitern gebildete Geflecht kann somit so angepasst werden, dass sich ein Leiterkonstrukt ergibt, dass insbesondere für eine günstige MR-Kompatibilität angepasst ist. Beispielsweise kann zumindest ein erster Leiter und/oder zumindest ein zweiter Leiter an einer jeweils zugeordneten Unterbrechungsstelle elektrisch unterbrochen sein, sodass ein sich wendelförmig um die Längsachse erstreckter Leiter an einer Stelle aufgetrennt ist.

Zum Auftrennen eines Leiters kann beispielsweise ein Laser-Schneidprozess verwendet werden. In einem Ausgangszustand liegt das Geflecht der Leiter zusammenhängend vor, wobei sich jeder Leiter entlang der gesamten Länge des Geflechts erstreckt und somit (in etwa) die gleiche Länge wie die übrigen Leiter aufweist. Zur Konfiguration einer Elektrodenleitung insbesondere mit Blick auf die MR-Kompatibilität bei einer bestimmten MR-Anregungsfrequenz können einzelne Leiter elektrisch miteinander verbunden und einzelne Leiter unterbrochen werden, sodass Zuleitungen für Elektrodenpole mit Stichleitungen zur Anpassung der effektiven elektrischen Länge der Zuleitungen verschaltet werden können.

In einer Ausgestaltung ist der zumindest eine Elektrodenpol ringförmig ausgebildet und erstreckt sich umfänglich um die Längsachse um das Geflecht herum. Der Elektrodenpol kann zur Herstellung der Elektrodenleitung auf das Geflecht aufgeschoben werden, wobei der Elektrodenpol beispielsweise durch Herstellung einer Schweiß- oder Lötverbindung mit einem darunter verlaufenden elektrischen Leiter elektrisch kontaktiert werden kann, um den Elektrodenpol an einen zugeordneten, eine Zuleitung ausbildenden Leiter anzuschließen. Sind mehrere Elektrodenpole vorhanden, wird jeder Elektrodenpol mit einem zugeordneten Leiter, der als Zuleitung dient, verbunden, wobei jeder als Zuleitung dienende Leiter wiederum mit einem weiteren, als Stichleitung zur Anpassung der effektiven elektrischen Länge dienenden Leiter verbunden werden kann.

In einer Ausgestaltung ist zumindest einigen Leitern der Mehrzahl von Leitern der Elektrodenleitung je zumindest eine Begleitfaser zugeordnet, die sich parallel zu dem jeweiligen Leiter erstreckt. Die Begleitfaser kann fest mit dem zugeordneten Leiter verbunden sein, sodass sich ein aus einem Leiter und einer Begleitfaser zusammengesetzter, wendelförmig gewundener Leitungsstrang ergibt.

Einem jeden Leiter kann hierbei eine einzelne Begleitfaser zugeordnet sein. Denkbar und möglich ist aber auch, dass ein Leiter zwischen zwei zugeordneten Begleitfasern eingefasst ist, wobei (betrachtet entlang der Längsachse) an jeder Seite des Leiters eine Begleitfaser angeordnet und beispielsweise mit dem Leiter verbunden ist.

Die Begleitfasern der Leiter sind vorzugsweise aus einem elektrisch isolierenden Material gefertigt. Solche Begleitfasern können beispielsweise aber auch elektrisch leitfähig sein oder einen elektrisch leitfähigen. von einer Isolierung umgebenen Kern aufweisen, um zum Beispiel eine elektrische Schirmung bereitzustellen.

In einer Ausgestaltung weist ein jeweiliger Leiter, gemessen radial zur Längsachse, eine erste Dicke auf, während die dem Leiter zugeordnete Begleitfaser eine zweite Dicke aufweist, die größer als die erste Dicke ist. Die Begleitfaser ist somit dicker als der zugeordnete Leiter, was bewirkt, dass die Begleitfaser einen Abstandshalter für den Leiter bereitstellt und insbesondere verhindert, dass die Leiter des Geflechts unmittelbar aneinander anliegen und sich gegenseitig mit Druck beaufschlagen. Über die Begleitfasern können die einzelnen Stränge des Geflechts aneinander abgestützt sein, sodass eine unmittelbare Anlage von Leitern aneinander verhindert ist. Durch die Begleitfasern wird somit ein mechanischer Schutz für die elektrischen Leiter der Elektrodenleitung bereitgestellt.

Die Leiter können farblich markiert sein, sodass einzelne Leiter des Geflechts voneinander unterschieden werden können. Zusätzlich oder alternativ können Begleitfasern der Leiter farblich markiert sein, sodass über die Begleitfasern eine Unterscheidbarkeit der einzelnen Leiter des Geflechts ermöglicht wird.

In einer Ausgestaltung weist die implantierbare Elektrodenleitung zumindest ein elektrisches Kontaktelement zum elektrischen Anschließen der implantierbaren Elektrodenleitung an ein aktives Gerät auf. Ein solches aktives Gerät kann zum Beispiel als Herzschrittmacher, CRT-Gerät, Defibrillator oder auch als Elektrophysiologie-Gerät ausgebildet sein. In diesem Fall ist die Elektrodenleitung - als Herzelektrodenleitung - insbesondere in das Herz eines Patienten zu implantieren. Die Elektrodenleitung kann aber auch als Neuroelektrodenleitung zur Neurostimulation im Rückenmark oder im Gehirn (sogenannte Spinal Chord Stimulation oder Deep Brain Stimulation) Verwendung finden.

In implantierter Stellung liegt die Elektrodenleitung mit ihren Elektrodenpolen an einem Stimulationsort im Patienten, zum Beispiel im Bereich des menschlichen Herzens oder im Bereich des Rückenmarks. Das aktive Gerät kann als implantierbares Gerät ebenfalls in den Patienten implantiert sein (zum Beispiel in Form eines Herzschrittmachers). Das aktive Gerät kann sich aber auch außerhalb des Patienten befinden.

Während ein Kontaktelement zum Beispiel an einem Stecker der Elektrodenleitung zum Verbinden mit dem aktiven Gerät vorzugsweise an einem proximalen Ende der Elektrodenleitung angeordnet ist, ist ein zugeordneter Elektrodenpol üblicherweise an einem zum Beispiel an einem Stimulationsort zu implantierenden, distalen Ende der Elektrodenleitung angeordnet. Das aus den Leitern gebildete Geflecht erstreckt sich vom proximalen Ende der Elektrodenleitung bis hin zum distalen Ende, wobei Leiter des Geflechts zum Ausbilden von Zuleitungen im Bereich des proximalen Endes mit zugeordneten Kontaktelementen und im Bereich des distalen Endes mit zugeordneten Elektrodenpolen elektrisch verbunden sind.

Die Aufgabe wird auch gelöst durch ein Verfahren zur Herstellung einer implantierbaren Elektrodenleitung, aufweisend folgende Schritte: Bereitstellen zumindest eines Elektrodenpols; Bereitstellen einer Mehrzahl von elektrischen Leitern, von denen zumindest einer mit dem zumindest einen Elektrodenpol elektrisch zu verbinden ist, wobei die Mehrzahl von Leitern zu einem entlang einer Längsachse erstreckten Geflecht miteinander verbunden ist, wobei zumindest ein erster Leiter der Mehrzahl von Leitern wendelförmig in eine erste Drehrichtung um die Längsachse und zumindest ein zweiter Leiter der Mehrzahl von Leitern wendelförmig in eine der ersten Drehrichtung entgegengesetzte, zweite Drehrichtung um die Längsachse gewunden ist; und Verbinden des zumindest einen Elektrodenpols mit zumindest einem Leiter der Mehrzahl von Leitern.

Die vorangehend für die Elektrodenleitung beschriebenen Vorteile und vorteilhaften Ausgestaltungen finden analog auch auf das Verfahren Anwendung, sodass auch auf das vorangehend Ausgeführte verwiesen werden soll.

Das Geflecht liegt in einem Ausgangszustand getrennt von den Elektrodenpolen der herzustellenden Elektrodenleitung vor und ist beispielsweise auf einen Innenschlauch geflochten, sodass sich das Geflecht um den Innenschlauch der Elektrodenleitung herum erstreckt. Zur Verbindung von Leitern des Geflechts mit Elektrodenpolen können die vorzugsweise ringförmig geformten Elektrodenpole auf das Geflecht geschoben werden und an einem vorbestimmten Ort - vorgegeben insbesondere durch einen vorbestimmten Abstand der Elektrodenpole zueinander - mit einem zugeordneten elektrischen Leiter des Geflechts verbunden werden.

Die Verbindung eines Elektrodenpols mit einem zugeordneten elektrischen Leiter, der als Zuleitung für den Elektrodenpol dienen soll, kann beispielsweise durch eine Schweiß- oder Lötverbindung hergestellt werden.

Beispielsweise kann ein Elektrodenpol eine Öffnung in seiner ringförmigen Mantelfläche aufweisen, über die eine Schweißverbindung mit einem unter dem Elektrodenpol gelegenen elektrischen Leiter hergestellt werden kann. Beispielsweise kann hierzu - nach Entfernung einer Isolierung des Leiters - ein Rand der Öffnung aufgeschmolzen werden, sodass aufgeschmolzenes Material des Elektrodenpols in den Bereich der Öffnung fließt und darüber eine elektrische Kontaktierung mit dem Leiter hergestellt wird.

Es sind darüber hinaus jedoch ganz unterschiedliche Verbindungsverfahren zur elektrischen Verbindung eines Elektrodenpols mit einem zugeordneten Leiter möglich, beispielsweise ein Laserschweißverfahren, ein Widerstandsschweißverfahren, ein Lötverfahren oder auch eine Verbindung mittels Klemmen.

Das Geflecht ist vorzugsweise aus Leitern gebildet, die die gleiche Länge aufweisen. Die Leiter erstrecken sich somit in einem Ausgangszustand entlang der gesamten Länge des Geflechts und können, zum Herstellen der Elektrodenleitung, mit zugeordneten Elektrodenpolen und Kontaktelementen und/oder miteinander elektrisch verbunden werden. Aus dem im Ausgangszustand aus Leitern einheitlicher Länge gebildeten Geflecht kann somit eine flexibel anpassbare Leiterstruktur für die Elektrodenpole zur Verbindung mit zugeordneten Kontaktelementen und zur Anpassung insbesondere mit Blick auf eine MR-Kompatibilität geschaffen werden.

Hierzu können einzelne Leiter des Geflechts auch elektrisch aufgetrennt werden, sodass zum Beispiel Stichleitungen gewünschter Länge an Zuleitungen geschaffen werden. Ein Leiter kann hierbei an einer oder mehreren Unterbrechungsstellen elektrisch aufgetrennt werden, sodass der Leiter elektrisch unterbrochen und Leitungsabschnitte verkürzter Länge geschaffen werden.

Nach Konfiguration der Leiter des Geflechts werden die Leiter des Geflechts vorzugsweise ummantelt, wobei das Geflecht zum Beispiel mit Kunststoff umspritzt oder eine äußere Ummantelung unter Verwendung eines Reflowprozesses geformt werden kann.

Zur Herstellung einer Äußeren Ummantelung mittels eines Reflowprozesses können beispielsweise Schlauchabschnitte über das an dem Innenschlauch angeordnete Geflecht geschoben werden, um diese Schlauchabschnitte sodann durch Aufschmelzen miteinander zu verbinden und somit eine zusammenhängende Ummantelung für die Elektrodenleitung zu schaffen. Die einzelnen Abschnitte können hierbei, in einer Ausgestaltung, unterschiedliche Steifigkeiten aufweisen, sodass die Elektrodenleitung in einem oder mehreren Abschnitten in flexibler Weise biegbar, in anderen Abschnitten hingegen weitestgehend starr ausgebildet sein kann.

Der der Erfindung zugrunde liegende Gedanke soll nachfolgend anhand der in den Figuren dargestellten Ausführungsbeispiele näher erläutert werden. Es zeigen:
- Fig. 1: eine Ansicht eines Ausführungsbeispiels einer Elektrodenleitung mit zu einem Geflecht geflochtenen elektrischen Leitern;
- Fig. 2: eine ausschnittsweise vergrößerte Ansicht im Ausschnitt X gemäß Fig. 1;
- Fig. 3: eine Ansicht eines Ausführungsbeispiels einer Elektrodenleitung mit einem an einem Innenschlauch angeordneten Geflecht von Leitern;
- Fig. 4: eine Querschnittansicht entlang der Linie A-A gemäß Fig. 3;
- Fig. 5: eine Ansicht eines anderen Ausführungsbeispiels einer Elektrodenleitung mit einem an einem Innenschlauch angeordneten Geflecht von Leitern;
- Fig. 6: eine Querschnittansicht entlang der Linie B-B gemäß Fig. 5;
- Fig. 7: eine Ansicht eines anderen Ausführungsbeispiels einer Elektrodenleitung mit einem Geflecht von Leitern; und
- Fig. 8: eine schematische Ansicht einer Elektrodenleitung.

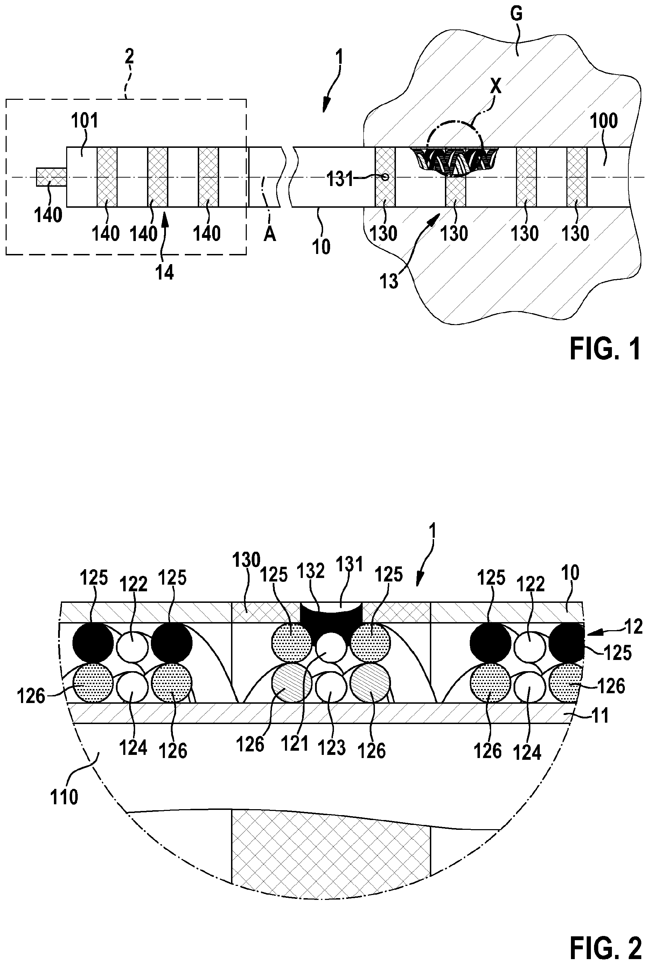

Fig. 1 zeigt eine Ansicht eines Ausführungsbeispiels einer Elektrodenleitung 1, die an einem proximalen Ende 101 mit einem aktiven Gerät 2 zu verbinden ist und mit einem distalen Ende 100 in Gewebe G, zum Beispiel im menschlichen Herzen, zu implantieren ist, um zum Beispiel eine Stimulation an einem gewünschten Stimulationsort zu bewirken.

Eine solche Elektrodenleitung 1 kann beispielsweise als Herzelektrodenleitung zur Implantation im menschlichen Herzen verwendet werden. Eine solche Elektrodenleitung 1 kann aber auch als Neuroelektrodenleitung ausgebildet und somit in das Rückenmark oder in das Gehirn eines Patienten implantiert werden.

Bei Verwendung als Herzelektrodenleitung kann das aktive Gerät 2 beispielsweise als Herzschrittmacher, CRT-Gerät, Defibrillator oder Elektrophysiologie-Gerät, zum Beispiel für eine Katheter-Ablation, ausgestaltet sein. Das aktive Gerät 2 kann, in einer Ausgestaltung, ebenfalls implantiert werden. Alternativ kann das aktive Gerät 2 auch außerhalb des menschlichen Körpers betrieben und somit außerhalb des menschlichen Körpers mit der Elektrodenleitung 1 verbunden sein.

Bei Verwendung als Neuroelektrodenleitung ist das aktive Gerät 2 zur Neurostimulation im Rückenmark oder im menschlichen Gehirn ausgebildet (sogenannte Spinal Chord Stimulation oder Deep Brain Stimulation).

Die Elektrodenleitung 1 weist eine Mehrzahl von im Bereich des distalen Endes 100 angeordneten Elektrodenpolen 130 auf, die eine Elektrodenpolanordnung 13 ausbilden und über die Stimulationsimpulse abgegeben und Signale detektiert werden können. Am proximalen Ende 101 der Elektrodenleitung 1 ist demgegenüber eine Kontaktanordnung 14 mit Kontaktelementen 140 zur Ausbildung eines (zum Beispiel nach der IS4-/DF4-Norm ausgebildeten) Steckers zur elektrischen Verbindung mit einem zugeordneten aktiven Gerät 2 angeordnet.

Innerhalb eines durch eine äußere Ummantelung gebildeten Außenschlauchs 10 sind elektrische Leiter eingefasst, die dazu dienen, die Kontaktelemente 140 elektrisch mit den Elektrodenpolen 130 zu verbinden und sich dazu entlang der Länge der Elektrodenleitung 1 innerhalb des Außenschlauchs 10 erstrecken.

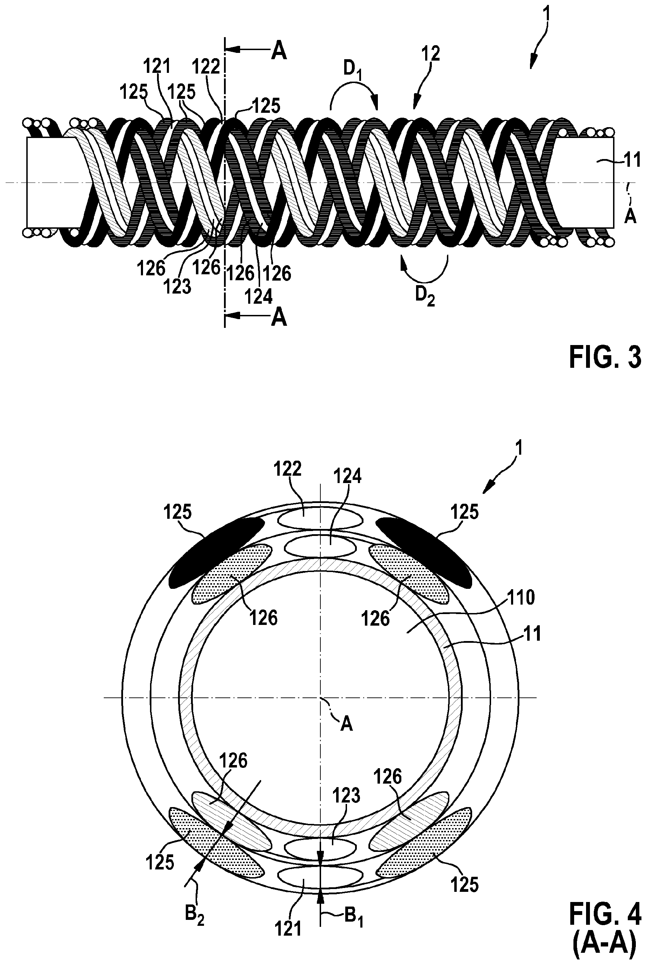

Bei der Elektrodenleitung 1 des Ausführungsbeispiels gemäß Fig. 1 sind elektrische Leiter 121-124, wie aus den Ansichten gemäß Fig. 2 bis 4 ersichtlich, zu einem Geflecht 12 miteinander verflochten. Erste elektrische Leiter 121, 122 erstrecken sich hierbei wendelförmig in eine erste Drehrichtung D1 (siehe Fig. 3) um eine Längsachse A, entlang derer sich die Elektrodenleitung 1 erstreckt. Zweite Leiter 123, 124 sind demgegenüber wendelförmig mit umgekehrter Drehrichtung D2 um die Längsachse A gewunden, wobei die Leiter 123, 124 abwechselnd übereinander und untereinander zu liegen kommen und somit ein zweilagiges Geflecht 12 an einem Innenschlauch 11 der Elektrodenleitung 1 ausbilden.

Die Leiter 121-124 des Geflechts 12 weisen jeweils einen elektrisch leitfähigen Kern auf, der durch eine isolierende Umhüllung eingehüllt ist, sodass die Leiter 121-124 gegeneinander elektrisch isoliert sind.

Auch bei implantierter Elektrodenleitung 1 sollen medizinische Untersuchungen ohne Einschränkung am Patienten möglich sein, insbesondere auch eine MRT-Untersuchung, gegebenenfalls sogar im Rahmen der Implantation zur Verifikation der Lage der Elektrodenleitung 1. Dabei ist eine übermäßige Erwärmung aufgrund einer Einkopplung eines elektromagnetischen Felds im Rahmen der MRT-Untersuchung zu vermeiden, um eine Verletzung eines Patienten auszuschließen.

Bei dem in Fig. 1 bis 4 dargestellten Ausführungsbeispiel sind insgesamt vier Leiter 121-124 zu einem Geflecht 12 miteinander verbunden und wendelförmig um den Innenschlauch 11 gewickelt. Die Leiter 121-124 verbinden bei dem dargestellten Ausführungsbeispiel die Kontaktelemente 140 der Kontaktanordnung 14 am proximalen Ende 101 der Elektrodenleitung 1 mit den Elektrodenpolen 130 der Elektrodenpolanordnung 13 am distalen Ende 100 der Elektrodenleitung 1. Durch Auswahl der Steigung der wendelförmig aufgewickelten Leiter 121-124 und somit durch Auswahl des Maschenabstands des Geflechts 12 kann die Länge der Leiter 121-124 so eingestellt werden, dass eine elektromagnetische Anregung bei einer vorbestimmten MR-Anregungsfrequenz wirkungsvoll verhindert ist.

Die Leiter 121-124 erstrecken sich entlang der Länge der Elektrodenleitung 1 und können vorzugsweise die gleiche Länge aufweisen.

Bei dem dargestellten Ausführungsbeispiel kann ein Elektrodenpol 130, wie aus Fig. 1 und 2 ersichtlich, an einem bestimmten axialen Ort mit einem zugeordneten Leiter 121 verbunden werden, indem beispielsweise eine Schweißverbindung zwischen dem Elektrodenpol 130 und dem Leiter 121 hergestellt wird. Dies kann beispielsweise durch ein sogenanntes Lochschlussschweißen erfolgen, im Rahmen dessen - nach Entfernung der Isolierung des Leiters 121 - eine Umrandung einer Öffnung 131 des Elektrodenpols 130 aufgeschmolzen wird, aufgeschmolzenes Material des Elektrodenpols 13 dadurch in den Bereich des Leiters 121 fließt und somit eine elektrische Kontaktierung herstellt, wie dies aus Fig. 2 ersichtlich ist.

Dadurch, dass die Elektrodenpole 130 ringförmig ausgebildet sind und sich die Leiter 121-124 zur Ausbildung des Geflechts 12 wendelförmig um den Innenschlauch 11 erstrecken, ist eine exakte axiale Positionierung der Elektrodenpole 130 möglich, um insbesondere einen vorgegebenen axialen Abstand der Elektrodenpole 130 zueinander einzustellen und einzuhalten. Die Elektrodenpole 130 werden hierzu so an dem Geflecht 12 positioniert und verdreht, dass die jeweilige Öffnung 131 eines Elektrodenpols 130 mit einem darunterliegenden, zugeordneten Leiter 121-124 fluchtet und somit eine Verbindung zu dem Leiter 121-124 hergestellt werden kann.

Das Geflecht 12 kann weitere Leiter aufweisen, die nicht (unmittelbar) mit einem Elektrodenpol 130 oder einem Kontaktelement 140 zu verbinden sind, sondern als Stichleitungen zur Verlängerung der elektrischen Länge der als Zuleitungen dienenden, mit den Elektrodenpolen 130 kontaktierten Leitern dienen.

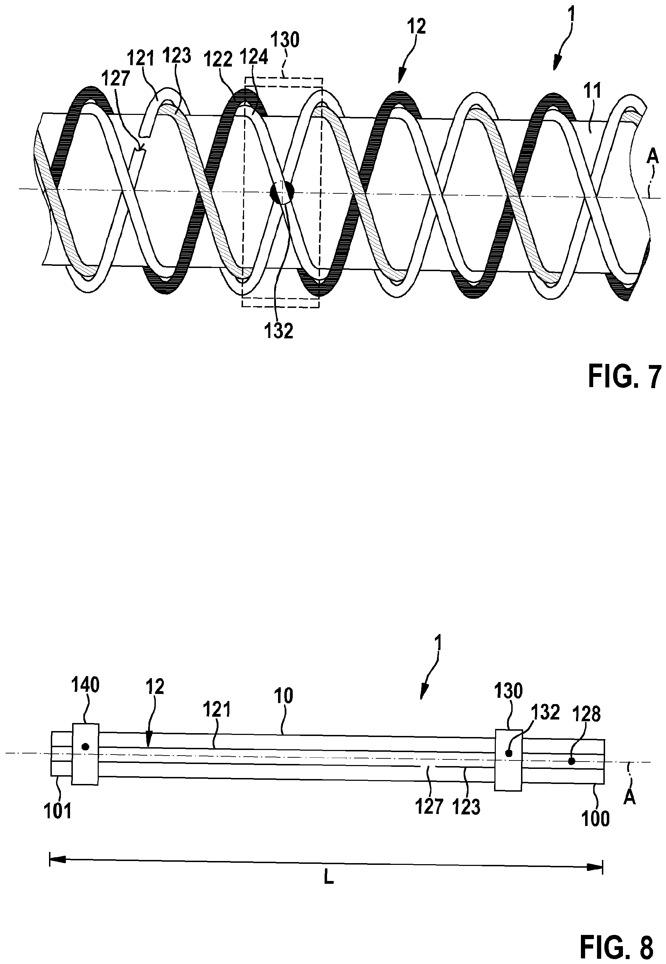

Dies ist schematisch in Fig. 8 dargestellt. So kann das Geflecht 12 aus (wendelförmig erstreckten, in Fig. 8 aus Gründen der vereinfachten Darstellung jedoch geradlinig gezeichneten) Leitern 121, 123 gebildet sein, die in entgegengesetzten Drehrichtungen D1, D2 um die Längsachse A der Elektrodenleitung 1 gewickelt sind, wobei zum Beispiel in die erste Drehrichtung D1 gewundene, erste Leiter 121 an je einer zugeordneten Verbindungsstelle 132 mit einem zugeordneten Elektrodenpol 130 elektrisch kontaktiert sind, entgegengesetzt in die zweite Drehrichtung D2 gewundene, zweite Leiter 123 demgegenüber an je einer zugeordneten Verbindungstelle 128 mit einem zugeordneten ersten Leiter 121 elektrisch verbunden sind.

Der Leiter 121 ist an der Verbindungstelle 132 mit dem zugeordneten Elektrodenpol 130 verbunden und erstreckt sich darüber hinaus bis zum distalen Ende 100 der Elektrodenleitung 1. Im Bereich des proximalen Endes 101 ist der Leiter 121 mit einem zugeordneten Kontaktelement 140 verbunden, erstreckt sich aber auch über das Kontaktelement 140 hinaus bis zum Ende der Elektrodenleitung 1. Ein Auftrennen eines als Zuleitung dienenden Leiters 121 ist nicht erforderlich, sodass sich sämtliche als Zuleitungen dienenden Leiter 121 über die gleiche, der Gesamtlänge der Elektrodenleitung 1 entsprechende Länge L erstrecken.

Die zweiten Leiter 123 können bei dem dargestellten Beispiel beispielsweise an einer oder an mehreren Unterbrechungsstellen 127 elektrisch aufgetrennt sein, sodass Leitungsabschnitte mit verkürzter Länge geschaffen sind.

Anzumerken ist hierzu, dass grundsätzlich ganz unterschiedliche Konfigurationen von als Zuleitungen dienenden Leitern und als Stichleitungen dienenden Leitern geschaffen werden können. So können insbesondere in die erste Drehrichtung D1 gewundene erste Leiter 121 und/oder in die zweite Drehrichtung D2 gewundene zweite Leiter 123 als Zuleitungen dienen, und entsprechend können in die zweite Drehrichtung D2 gewundene zweite Leiter 123 und/oder in die erste Drehrichtung D1 gewundene erste Leiter 121 als Stichleitungen verwendet werden.

Durch die Verwendung von Leitern 121, 123, die sich entlang der gesamten Länge L der Elektrodenleitung 1 erstrecken, als Zuleitungen oder als Stichleitungen und durch elektrisches Verbinden eines als Zuleitung dienenden Leiters mit einem anderen, als Stichleitung dienenden Leiter kann die elektrische Länge einer Zuleitung verdoppelt werden, wobei auch denkbar und möglich ist, mehr als zwei Leiter miteinander zu verbinden, sodass die effektive elektrische Länge einer Zuleitung auch über die doppelte Länge der Elektrodenleitung 1 hinaus verlängert werden kann.

Bei dem Ausführungsbeispiel gemäß Fig. 1 bis 4 sind einem jeden Leiter 121-124 zwei Begleitfasern 125, 126 zugeordnet, die - betrachtet entlang der Längsachse A der Elektrodenleitung 1 - beidseits des jeweils zugeordneten Leiters 121-124 angeordnet sind und den jeweiligen Leiter 121-124 somit zwischen sich einfassen. Wie aus der Schnittansicht gemäß Fig. 4 ersichtlich, weisen die Begleitfasern 125, 126 jeweils eine Dicke B2 (gemessen radial im Querschnitt quer zur Längsachse A) auf, die größer ist als die Dicke B1 der zugeordneten Leiter 121-124. Dies bewirkt, dass die Leiter 121-124 mechanisch nicht unmittelbar miteinander in Anlage sind, sondern über die Begleitfasern 125, 126 zueinander abgestützt sind, was die Leiter 121-124 vor Beschädigung schützt.

Die Begleitfasern 125, 126 können fest mit den jeweils zugeordneten Leitern 121-124 verbunden sein. Denkbar und möglich ist aber auch, die Begleitfasern 125, 126 lose neben den Leitern 121-124 zu verlegen.

Zur Herstellung wird der Innenschlauch 11 auf einen beispielsweise starr ausgebildeten Kern geschoben, und die Leiter 121-124 werden zum Beispiel unter Verwendung einer Flechtmaschine um den Innenschlauch 11 zur Ausbildung des Geflechts 12 geflochten. Die Begleitfasern 125, 126 werden hierbei zusammen mit den Leitern 121-124 geflochten.

Nach dem Flechten des Geflechts 12 können die einzelnen Leiter 121-124 mit zugeordneten Elektrodenpolen 130 der Elektrodenpolanordnung 13 und mit Kontaktelementen 140 der Kontaktanordnung 14 elektrisch verbunden werden. Zudem können einzelne Leiter 121-124 miteinander kontaktiert werden, um Stichleitungen zur Verlängerung der effektiven elektrischen Länge einer Zuleitung zu schaffen. Die Stichleitungslänge kann hierbei durch Auftrennen einzelner Leiter 121-124 jeweils in gewünschter Weise angepasst werden.

Nach der Konfiguration des Geflechts 12 zur elektrischen Verbindung der Elektrodenpole 130 mit den Kontaktelementen 140 wird der Außenschlauch 10 an dem Geflecht 12 gebildet. Dies kann beispielsweise durch Umspritzen erfolgen. Alternativ kann ein Reflowprozess zum Einsatz kommen, im Rahmen dessen Schlauchabschnitte auf das Geflecht 12 geschoben und durch Aufschmelzen zur Ausbildung einer äußeren Umhüllung verbunden werden. Die Elektrodenpole 130 und die Kontaktelemente 140 bleiben hierbei von außen zugänglich und werden nicht umhüllt.

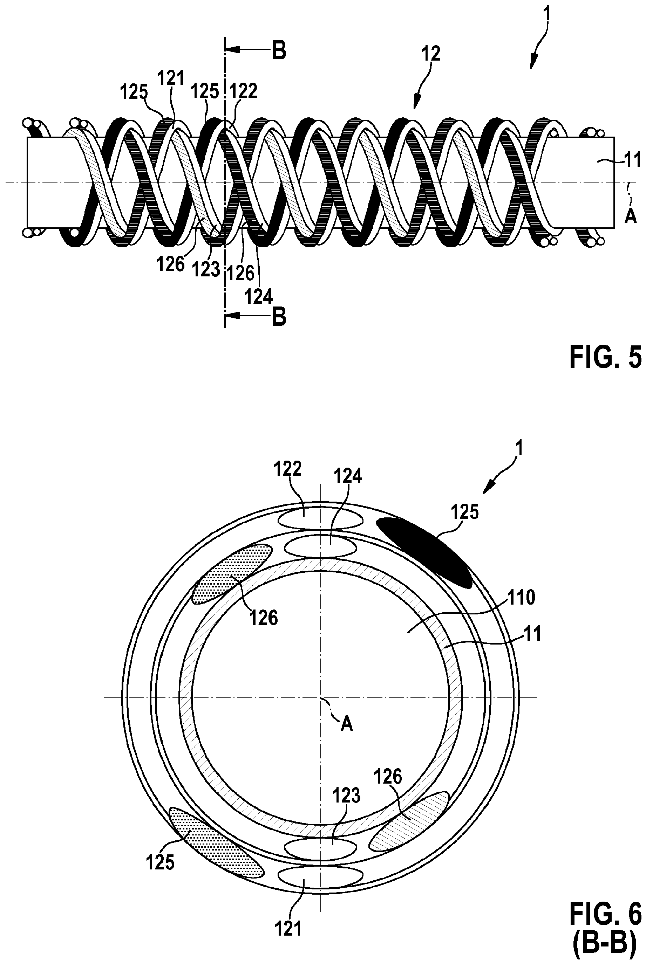

Bei einem in Fig. 5 und 6 dargestellten Ausführungsbeispiel ist, im Vergleich zum Ausführungsbeispiel gemäß Fig. 1 bis 4, das Geflecht 12 aus Leitern 121-124 gebildet, denen jeweils nur eine Begleitfaser 125, 126 zugeordnet ist. Ansonsten ist das Ausführungsbeispiel gemäß Fig. 5 und 6 funktionsgleich dem Ausführungsbeispiel gemäß Fig. 1 bis 4, sodass auch auf die vorangehenden Ausführungen verwiesen werden soll.

Bei einem in Fig. 7 dargestellten Ausführungsbeispiel weisen Leiter 121-124 des Geflechts 12 der Elektrodenleitung 1 keine Begleitfasern auf. Gestrichelt eingezeichnet ist in Fig. 7 ein Elektrodenpol 130, der mit einem Leiter 124 als Zuleitung zu verbinden ist. Ein Leiter 121 kann demgegenüber als Stichleitung dienen und ist elektrisch an einer Unterbrechungsstelle 127 aufgetrennt. Der Leiter 121 kann hierbei an einer Verbindungstelle 132, an der der Elektrodenpol 130 mit dem Leiter 124 elektrisch kontaktiert ist, auch mit dem Leiter 124 kontaktiert sein, sodass über die Verbindungstelle 132 eine elektrische Verbindung der Leiter 121, 124 miteinander und zudem des Leiters 124 mit dem Elektrodenpol 130 geschaffen wird.

Bei den dargestellten Ausführungsbeispielen sind die Leiter 121-124 zu einem Geflecht 12 zweilagig miteinander verflochten derart, dass sich die Leiter 121-124 abwechselnd übereinander und untereinander erstrecken. Das Geflecht 12 ist somit in einer Geflechtebene gefertigt und dabei (in seiner Grundform) schlauchförmig um die Längsachse A der Elektrodenleitung 1 erstreckt.

Denkbar ist in anderer Ausgestaltung auch, ein Geflecht 12 mit mehreren Geflechtebenen auszubilden, wobei jede Geflechtebene zweilagig durch abwechselnd übereinander und untereinander erstreckte Leiter gefertigt ist. Auf diese Weise kann die Anzahl der Leiter der Elektrodenleitung 1 erhöht werden.

Der der Erfindung zugrunde liegende Gedanke ist nicht auf die vorangehend geschilderten Ausführungsbeispiele beschränkt, sondern lässt sich auch in andersgearteten Ausführungsformen verwirklichen.

Eine Elektrodenleitung hier beschriebenen Art kann grundsätzlich in ganz unterschiedlichen Anwendungen mit jeweils zugeordneten aktiven Geräten, zum Beispiel implantierbaren aktiven Geräten oder auch extern eines Patienten zu verwendenden aktiven Geräten, Verwendung finden.

Durch die Verwendung eines Geflechts, das durch Leiter der Elektrodenleitung ausgebildet ist, ergibt sich eine günstige Verlegung der Leiter bei guter Ausnutzung des zur Verfügung stehenden Bauraums und flexibler Konfigurierbarkeit der Elektrodenleitung insbesondere mit Blick auf eine MRT-Kompatibilität.

Zur Herstellung des Geflechts können eine Vielzahl von Leitern gleichzeitig in vorteilhafter Weise an einem Innenschlauch der Elektrodenleitung geflochten werden, sodass sich eine schlauchförmige Grundform ergibt, die flexibel in ihrer Form anpassbar ist und zudem durch Verbindung der Leiter mit Elektrodenpolen, Kontaktelementen und miteinander sowie durch Anpassung der Längen der Leiter durch lokales Auftrennen elektrisch konfiguriert werden kann.

Die Elektrodenleitung kann grundsätzlich eine beliebige Anzahl von Leitern aufweisen, zum Beispiel eine Anzahl zwischen zwei und mehreren hundert Leitern, die gemeinsam das Geflecht ausbilden.

### Liste der Bezugszeichen

- 1: Implantierbare Elektrode
- 10: Außenschlauch
- 100, 101: Ende
- 11: Innenschlauch
- 110: Lumen
- 12: Geflecht
- 121-124: Leiter
- 125, 126: Begleitfaser
- 127: Unterbrechungsstelle
- 128: Verbindungsstelle
- 13: Polanordnung
- 130: Elektrodenpol
- 131: Öffnung
- 132: Verbindungsstelle
- 14: Kontaktanordnung
- 140: Kontaktelement
- 2: Aktives Gerät
- A: Längsachse
- B1, B2: Dicke
- D1, D2: Drehrichtung
- G: Gewebe
- L: Länge

## Patentansprüche

1. Implantierbare Elektrodenleitung (1), mit
zumindest einem Elektrodenpol (130) und
einer Mehrzahl von elektrischen Leitern (121-124), von denen zumindest einer mit dem zumindest einen Elektrodenpol (130) elektrisch verbunden ist,
**dadurch gekennzeichnet,**
**dass** die Mehrzahl von Leitern (121-124) zu einem entlang einer Längsachse (A) erstreckten Geflecht (12) miteinander verbunden ist, wobei zumindest ein erster Leiter (121, 122) der Mehrzahl von Leitern (121-124) wendelförmig in eine erste Drehrichtung (D1) um die Längsachse (A) und zumindest ein zweiter Leiter (123, 124) der Mehrzahl von Leitern (121-124) wendelförmig in eine der ersten Drehrichtung (D1) entgegen gesetzte, zweite Drehrichtung (D2) um die Längsachse (A) gewunden ist.

2. Implantierbare Elektrodenleitung (1) nach Anspruch 1, **dadurch gekennzeichnet, dass** das Geflecht (12) eine Länge (L) aufweist, wobei die Mehrzahl von Leitern (121-124) entlang der Länge (L) des Geflechts (12) erstreckt ist.

3. Implantierbare Elektrodenleitung (1) nach Anspruch 1 oder 2, **dadurch gekennzeichnet, dass** die Mehrzahl von Leitern (121-124) an einem Innenschlauch (11) angeordnet und um den Innenschlauch (11) herum gewunden ist.

4. Implantierbare Elektrodenleitung (1) nach einem der Ansprüche 1 bis 3, **dadurch gekennzeichnet, dass** der zumindest eine erste Leiter (121, 122) an einer ersten Verbindungsstelle (132) elektrisch mit dem zumindest einen Elektrodenpol (130) verbunden ist und der zumindest eine zweite Leiter (123, 124) an einer zweiten Verbindungsstelle (128) mit dem zumindest einen ersten Leiter (121, 122) elektrisch verbunden ist.

5. Implantierbare Elektrodenleitung (1) nach einem der vorangehenden Ansprüche, **dadurch gekennzeichnet, dass** der zumindest eine erste Leiter (121, 122) und/oder der zumindest eine zweite Leiter (123, 124) zumindest eine Unterbrechungsstelle (127) aufweisen, an der der zumindest eine erste Leiter (121, 122) und/oder der zumindest eine zweite Leiter (123, 124) elektrisch unterbrochen sind.

6. Implantierbare Elektrodenleitung (1) nach einem der vorangehenden Ansprüche, **dadurch gekennzeichnet, dass** der zumindest eine Elektrodenpol (130) ringförmig ausgebildet ist und umfänglich um die Längsachse (A) um das Geflecht (12) herum erstreckt ist.

7. Implantierbare Elektrodenleitung (1) nach einem der vorangehenden Ansprüche, **dadurch gekennzeichnet, dass** der zumindest eine Elektrodenpol (130) an einer Verbindungsstelle (132) mit einem darunter verlaufenden Leiter der Mehrzahl von Leitern (121-124) elektrisch verbunden ist.

8. Implantierbare Elektrodenleitung (1) nach einem der vorangehenden Ansprüche, **dadurch gekennzeichnet, dass** zumindest einigen Leitern der Mehrzahl von Leitern (121-124) je zumindest eine Begleitfaser (125, 126) zugeordnet ist, die sich parallel zu dem jeweiligen Leiter erstreckt.

9. Implantierbare Elektrodenleitung (1) nach Anspruch 8, **dadurch gekennzeichnet, dass** die Begleitfaser (125, 126) aus einem elektrisch isolierenden Material gefertigt ist.

10. Implantierbare Elektrodenleitung (1) nach Anspruch 8 oder 9, **dadurch gekennzeichnet, dass** der jeweilige Leiter, gemessen radial zur Längsachse (A), eine erste Dicke (B1) und die zugeordnete, zumindest eine Begleitfaser (125, 126) eine zweite Dicke (B2) aufweisen, wobei die zweite Dicke (B2) größer ist als die erste Dicke (B1).

11. Implantierbare Elektrodenleitung (1) nach einem der vorangehenden Ansprüche, **gekennzeichnet durch** zumindest ein elektrisches Kontaktelement (140) zum elektrischen Anschließen der implantierbaren Elektrodenleitung (1) an ein aktives Gerät (2).

12. Verfahren zur Herstellung einer implantierbaren Elektrodenleitung (1), aufweisend:
Bereitstellen zumindest eines Elektrodenpols (130) und
Bereitstellen einer Mehrzahl von elektrischen Leitern (121-124), von denen zumindest einer mit dem zumindest einen Elektrodenpol (130) elektrisch zu verbinden ist, wobei die Mehrzahl von Leitern (121-124) zu einem entlang einer Längsachse (A) erstreckten Geflecht (12) miteinander verbunden ist, wobei zumindest ein erster Leiter (121, 122) der Mehrzahl von Leitern (121-124) wendelförmig in eine erste Drehrichtung (D1) um die Längsachse (A) und zumindest ein zweiter Leiter (123, 124) der Mehrzahl von Leitern (121-124) wendelförmig in eine der ersten Drehrichtung (D1) entgegen gesetzte, zweite Drehrichtung (D2) um die Längsachse (A) gewunden ist, und
Verbinden des zumindest einen Elektrodenpols (130) mit zumindest einem Leiter der Mehrzahl von Leitern (121-124).

13. Verfahren nach Anspruch 12, **dadurch gekennzeichnet, dass** der zumindest eine Elektrodenpol (130) eine Öffnung (131) aufweist, über die der zumindest eine Elektrodenpol (130) mit zumindest einem Leiter der Mehrzahl von Leitern (121-124) verbunden wird.

14. Verfahren nach Anspruch 12 oder 13, **dadurch gekennzeichnet, dass** das Geflecht (12) in einem Ausgangszustand eine Länge (L) aufweist, wobei die Mehrzahl von elektrischen Leitern (121-124) entlang der Länge (L) des Geflechts (12) erstreckt ist.

15. Verfahren nach einem der Ansprüche 12 bis 14, **dadurch gekennzeichnet, dass** zumindest ein Leiter der Mehrzahl von Leitern (121-124) an einer Unterbrechungsstelle (127) elektrisch unterbrochen wird.
